# EUROPÄISCHE PATENTSCHRIFT

(11) **EP 1 935 359 B1**
(45) Veröffentlichungstag und Bekanntmachung des Hinweises auf die Patenterteilung: **05.01.2011**
(21) Anmeldenummer: 07122696.3
(22) Anmeldetag: 10.12.2007
(51) Int. Cl.: A61B 17/70, A61B 17/80

(54) **Orthopädisches Schraubenverschlusssystem**
Orthopaedic screw lock system
Système de fermeture à vis orthopédique

(30) Priorität: 19.12.2006 DE 202006019220 U
(43) Veröffentlichungstag der Anmeldung: 25.06.2008
(73) Patentinhaber: Zrinski AG, 78573 Wurmlingen (DE)
(72) Erfinder: Altermann, Frank, 78532, Tuttlingen (DE); Reiter, Zoran, 78576, Emmenigen-Liptingen (DE); Eckhof, Stephan, 78604, Weilheim-Rietheim (DE); Maier, Rolf, 78576, Liptingen (DE); Zrinski, Josef, 78573, Wurmlingen (DE); Zrinski, Davor, 78604, Rietheim-Weilheim (DE)

(56) Entgegenhaltungen:
- EP-A- 1 649 819
- WO-A-2004/084701
- WO-A-2005/018472
- WO-A-2007/014192
- DE-U1- 20 309 361

## Beschreibung

### Technisches Gebiet

Die vorliegende Erfindung betrifft ein Schraubenverschlusssystem, das aus mindestens einer Schraube sowie mindestens einer Platte mit mindestens einer Durchgangsbohrung besteht. Die Schraube selbst besteht aus einem Schraubenkopf und einem Schraubenschaft, wobei zumindest der Schraubenkopf mit einem Gewinde versehen ist. Die Schraube ist polyaxial in die Durchgangsbohrung einführbar. Dies bedeutet, dass die Schraube nicht senkrecht zur Platte, sondern schräg zur Platte einführbar und mit der Platte verriegelbar ist. Eine kraft- oder eine kraft- und formschlüssige Verbindung zwischen der Schraube und Platte entsteht.

### Definitionen

### Durchgangsbohrung

Als Durchgangsbohrung sind solche Bohrungen definiert, die sich von der Oberfläche einer Platte bis zur Unterseite der Platte erstrecken. Sie kann zylindrisch oder konisch ausgebildet sein. In der Regel ist sie mit einem Bohrer oder einem Fräser herstellbar.

### Durchgangsöffnung

Als Durchgangsöffnung sind solche Öffnungen definiert, die zur Aufnahme einer Schraube mit Schraubenschaft und Schraubenkopf geeignet sind. In der Durchgangsöffnung geht der Schraubenkopf mit dem übrigen Teil der Platte die kraft- und formschlüssige Verbindung ein. Die Durchgangsöffnung entsteht in der Regel erst durch zwei oder mehr Durchgangsbohrungen, die derart zueinander angeordnet sind, dass diese sich schneiden. Zur Herstellung einer Durchgangsöffnung wird zunächst mit einer Zentralbohrung begonnen und ausgehend von dieser werden in regelmässigen Abständen weitere Durchgangsbohrungen ausgeführt, so dass in Draufsicht eine klee- oder blumen- beziehungsweise blätterartige Konstruktion entsteht.

### Schnittlinie

Schneiden sich mehrere Durchgangsbohrungen, so entstehen im Bereich der Durchgangsöffnungen Schnittlinie, die sich über die Dicke der Platterstrecken. Die Schnittlinien erstrecken sich somit in die Tiefe der Durchgangsöffnung. Vorzugsweise sind sie derart ausgebildet, dass sie sich in Richtung der Längsausdehnung der Durchgangsbohrung erstrecken. Die Schnittlinien sind Elemente, die mit dem Schraubenkopf zusammenwirken, um eine kraft- und formschlüssige Verbindung durch plastisches Umformen herbeizuführen.

### Schnittfläche

Aus den zuvor definierten Schnittlinien werden dann Schnittflächen, wenn die Durchgangsbohrungen und /oder die Zentralbohrung konisch gestaltet sind. Die Schnittflächen sind Elemente, die mit dem Schraubenkopf zusammenwirken, um eine kraft- und formschlüssige Verbindung durch plastisches Umformen herbeizuführen. Nachfolgend wird der Einfachheit halber nur von Schnittlinien gesprochen. Sofern nicht anders ausgeführt, wird aber auch darunter die Kombination aus Schnittlinien und Schnittflächen oder nur die Schnittflächen verstanden.

### Stand der Technik

Schrauben und Platten, insbesondere Knochenplatten der vorstehenden Art bilden in der Regel ein Fixierungssystem und werden vorwiegend dafür verwendet, Knochenfrakturen mechanisch zu stabilisieren. Dieses Fixierungssystem besteht aus einer Platte, die mit mindestens einer Durchgangsbohrung, in der Regel jedoch mehreren Durchgangsbohrungen versehen ist, wobei die Durchgangsbohrungen zur Aufnahme jeweils einer Schraube ausgebildet sind. Die Schraube selbst besteht aus einem Schraubenkopf und aus einem Schraubenschaft und weist an dem Schraubenschaft ein Gewinde auf, das dazu geeignet ist, in einen Knochen eingedreht zu werden. Der Schraubenkopf selbst weist ebenfalls ein Gewinde auf, der im montierten Zustand des Fixierungssystems mit der Platte in der jeweiligen Durchgangsbohrung zusammenwirkt

Orthopädische Fixationseinrichtungen sind sowohl innerhalb als auch ausserhalb des Körpers verwendbar. Sie bestehen aus einer plattenförmigen Ausbildung, die sich beispielsweise über eine Bruchstelle (Fraktur) hinweg erstreckt. Zudem sind Befestigungsmittel vorgesehen, die beispielsweise als Schrauben, Bolzen, Nägel oder Stifte ausgebildet sind. Beispielsweise können sogenannte Knochenplatten an einem Knochen mittels Knochenschrauben befestigt werden, indem letztere durch Durchgangsbohrungen gesteckt und im Knochen verschraubt werden. Um zu verhindern, dass diese Schrauben sich insbesondere bei Krafteinwirkung lösen, sind aus dem Stand der Technik eine Vielzahl von Mechanismen bekannt, die verhindern, dass ein Loslösen der Schraube und damit ein Loslösen der plattenförmigen Ausbildung möglich ist. Ferner sind Lösungsansätze bekannt, die es ermöglichen, zwischen der Platte und der Schraube eine kraftschlüssige Verbindung herzustellen, bei der die Schraube nicht senkrecht zur Platte ausgerichtet ist. Eine Schrägstellung einer solchen Schraube ist oft dann gewünscht, wenn beispielsweise aufgrund der Knochenstruktur kein Eindrehen einer Schraube senkrecht zur Platte möglich ist.

So sind beispielsweise aus der US 4484570 (SYNTHES LTD (US)) 27.11.1984 sogenannte Expansionskopfschrauben bekannt. Eine einmal in eine Platte eingedrehte Kopfschraube wird mit einem Zusatzschraubelement derart gespreizt, dass durch das Eindrehen der zusätzlichen Schraube eine Verkeilung innerhalb der Platte stattfindet, so dass eine Fixierung der Schraube an der Platte erfolgt.

Eine weitere Ausführungsform aus dem Stand der Technik, wie sie beispielsweise in der US 5954722 (DEPUY ACROMED INC (US)) 21.09.1999 aufgeführt ist zeigt, eine ausgebildete Platte, in die polyaxial eine Schraube eindrehbar ist. Die Polyaxialität hat unter anderem den Vorteil, dass die Einschraubrichtungen nicht mehr durch die Platte selbst vorgegeben sind. Dadurch ist es möglich, je nach Beschaffenheit des Materials, eine Schraube nicht unbedingt senkrecht zu der Plattenlängserstreckung in einen Knochen einzuschrauben. Hierfür ist vorgesehen, dass die Durchgangsbohrungen zusätzlich mit einem kugeligen Element versehen sind, das innerhalb der Durchgangsbohrung zumindest teilweise drehbar gelagert ist. Dieses kugelige Element ist unverlierbar innerhalb der Durchgangsbohrung angeordnet und wirkt mitdem Schraubenkopf der einzudrehenden Schraube zusammen. Je nach Lage orientiert sich das kugelige Element und stellt somit mit dem Schraubenkopf und der Platte eine kraft- und formschlüssige Verbindung her.

Eine weitere polyaxiale Ausführung einer Platte, die mit einer speziell ausgebildeten Schraube zusammenwirkt, ist in der US 20050165400 A (FERNANDEZ ALBERTO A, UY) 26.01.2004 dargestellt. Das orthopädische Fixationssystem weist eine Platte auf, die ebenfalls ein oder mehrere Durchgangsbohrungen aufweist. Ferner ist eine Schraube vorgesehen, die aus einem Schraubenkopf und einem Schraubenschaft besteht. Der Schraubenkopf ist speziell ausgebildet und weist ein Gewinde auf, das mit zumindest teilweise in den Durchgangsbohrungen vorhandenen Gewinden zusammenwirkt.

Um ein Einschrauben schräg (und damit nicht senkrecht) zur Längserstreckung der Platte zu ermöglichen, ist vorgesehen, die Durchgangsbohrung speziell auszubilden. Die Durchgangsbohrungen weisen im Querschnitt eine sanduhrartigen Form auf. Dies bedeutet, dass - in Einschraubrichtung gesehen - sich der Durchmesser der Durchgangsbohrung von einem weiten Durchmesser ausgehend zunächst verjüngt, bis eine definierte Ebene erreicht ist. Von dieser Ebene ausgehend weitet sich der Durchmesser der Durchgangsbohrung wieder auf.

Die Durchgangsbohrung, die in dieser Weise ausgebildet ist, weist ein Gewinde auf, das mit dem Gewinde des Schraubenkopfes zusammenwirkt. Das Gewinde ist besonders ausgebildet und als sogenanntes Schneidgewinde dargestellt. Dies bedeutet, dass beim Eindrehen des Schraubenkopfes ein mechanischer Schneidevorgang zwischen dem besagten Gewinde und dem Schraubenkopf stattfindet. Um diesen Schneidevorgang zu verstärken und damit ein Verkeilen des Schraubenkopfes innerhalb der Durchgangsbohrung zu erreichen, damit ein ungewolltes Lösen zu einem späteren Zeitpunkt von Platte und Schraube nicht mehr möglich ist, sind innerhalb der Durchgangsbohrung vorzugsweise aus einem anderen Material bestehende Schneidelemente vorgesehen. Diese Schneidelemente sind im Umfang der Durchgangsbohrung eingelassen und führen beim Eindrehen der Schraube in die Durchgangsbohrung eine Verformung herbei. Durch die kugelige Ausgestaltung des Schraubenkopfes ist es möglich, eine Vielzahl von Winkelgraden, die von der Senkrechten zur Längserstreckung der Platte abweichen, zu wählen.

Aus der DE 202004015912U (AESCULAP AG ) 09.12.2004 ist ebenfalls ein Fixationssystem bekannt, das im wesentlichen aus einer Platte und einer Knochenschraube besteht. Die Knochenschraube selbst weist einen mit einer Längsachse definieren Schaft und einen Schraubenkopf aus, welcher mit einer Knochenschraubenaufnahme eine Knochenplatte in Eingriff bringbar ist. Zusätzlich ist ein Sicherungselement zum Sichern der Verbindung der Knochenschraube und der Knochenplatte vorgesehen, wobei die Knochenschraube von einer Eingriffssteltung, in welcher die Knochenschraube an der Knochenplatte gehalten ist, in eine Lösestellung bringbar ist.

Die Durchgangsöffnungen innerhalb der Knochenplatte sind vorzugsweise oval gestaltet, sodass eine polyaxiale Aufnahme der Knochenschraube möglich ist. Sie weisen an ihren Wandungen Gewinde auf, die mit dem Gewinde der Knochenschraube beziehungsweise dem Schraubenkopf in Eingriff bringbar sind. Durch Verklemmen der Knochenschraube mit der Knochenplatte wird eine Sicherung der Knochenschraube erreicht. Die Herstellung der Durchgangöffnungen wird derart realisiert, dass durch Fräsen eine ovale Durchgangöffnung bereitgestellt wird, die Wandungen aufweist, die senkrecht zur Längserstreckung der Knochenplatte ausgebildet sind. Zusätzlich weisen die Wandungen Gewindegänge auf. Die Herstellung daher ist aufwendig und komplex. Die Knochenschraube selbst muss speziell ausgebildet sein und über den speziellen Sicherungsmechanismus verfügen.

Aus der WO 2004/084701 A (SWISS ORTHOPEDIC SOLUTIONS SA (CH); YOUNG ROBERT ALLAN (US)) 07.10.2004 ist eine Knochenplatte bekannt, die eine longitudinale Erstreckung aufweist. Die Knochenplatte selbst weist mehrere Durchgangsöffnungen auf, die durch zwei versetzt zueinander ausgestalteten Durchgangsbohrungen gebildet wird. Die Durchgangsbohrungen sind derart angeordnet, dass deren Mittelpunkt auf der Mittelachse der jeweiligen Knochenplatte angeordnet ist und um einen definierten Abstand voneinander angeordnet sind. Die beiden Durchgangsbohrungen schneiden sich derart, dass in Draufsicht eine achtförmige Ausgestaltung erreicht wird. Das bedeutet, dass zwischen den beiden Durchgangsbohrungen, die die Durchgangsöffnungen bilden, eine Verengung vorgesehen ist. Beide Durchgangsbohrungen weisen im unteren Bereich, dass heisst, dem den Knochen zugewandten Bereich Gewindegänge auf, die zueinander unterschiedlich sind. Auf der dem Knochen abgewandten Seite, dass heisst, auf der Oberseite ist der Öffnungsbereich der Durchgangsbohrung und damit auch der Durchgangsöffnung wesentlich grösser als der wie er im unteren Bereich vorgesehen ist.

Bei besonderen Ausführungsbeispielen sind die im unteren Bereich der Knochenplatte vorgesehenen Gewindegänge in einem Winkel zueinander angeordnet oder aber schräg.

Wie auch beim übrigen Stand der Technik ist es notwendig, die Knochenplatte in unterschiedlichen Bearbeitungszyklen herzustellen. Insbesondere gestaltet es sich als aufwendig, im unteren Bereich der Knochenplatte, dass heisst dem den Knochen zugewandten Bereich unterschiedliche Gewindegänge anzuordnen, die dann mit dem Kopf der Knochenschraube, der ebenfalls ein Gewinde aufweist, zusammenwirken können. Eine freie polyaxiale Anordnung der Knochenschraube ist damit nicht möglich, da sie beim Eindrehen unweigerlich mit dem Gewinde zusammenwirkt und dann die vorgegebene Richtung auch einnimmt.

Auch die DE 20321245U (SYNTHES GMBH (CH)) 14.06.2006 zeigt eine Knochenplatte mit einer dem Knochen zugewandten Unterseite und Oberseite und mehreren die Unterseite mit der Oberseite verbindenden Durchgangsbohrungen, die jeweils eine zentrale Lochachse, eine Innenmantelfläche und einen Gewindegang aufweisen. Die Durchgangsöffnung wird bei einem besonderen Ausführungsbeispiel durch eine zentrale Durchgangsbohrung mit einer die zentrale Durchgangsbohrungen am Umfang der Durchgangsbohrung angeordneten Durchgangsbohrungen, die im Winkel von jeweils 90° voneinander beanstandet sind, ausgeführt. Dadurch ist eine polyaxiale Anordnung der jeweiligen Knochenschrauben, deren Kopf sich in die jeweilige Durchgangsbohrung einfügt, gegeben. Die jeweiligen Durchgangsbohrungen weisen wie zuvor bereits beschrieben ebenfalls Gewindegänge auf.

Aus diesem Grund ist auch die Herstellung sehr komplex und die polyaxiale Richtung der jeweiligen Knochenschraube ist aufgrund der entsprechenden Vorgaben der Durchgangsbohrungen nicht frei wählbar.

Zudem ist keine Sicherungsmöglichkeit der jeweiligen Knochenschraube gegeben, sodass ein ungewolltes Lösen jederzeit möglich ist, wodurch die Gefahr gegeben ist, dass die Funktion der jeweiligen Knochenplatte vollständig ausser Funktion gerät.

Aus der EP 1 649819 A (MAIER CHRISTIAN (AT)) 26.04.2006 ist eine Knochenplatte mit wenigstens einer Durchgangsöffnung für eine Knochenschraube bekannt, wobei die Durchgangsöffnung verformbare Mittel aufweist, die zum winkelstabilen Festlegen der Knochenschraube geeignet sind. Diese Mittel zeichnen sich durch Vorsprünge innerhalb der Durchgangsöffnung aus, die im Wesentlichen parallel zur Längserstreckung der Knochenplatte ausgerichtet sind und eine gegenüber der Dicke der Knochenplatte wesentlich geringere Dicke aufweisen. Die Vorsprünge sind in der Mitte der Dicke der Durchgangsöffnung angeordnet.

Aus der WO 2005018472 A1 (MATHYY MEDIZINALTECHNIK AG [CH], SCHNEIDER, ROLF [CH]) 03.03.2005 ist eine Knochenplatte dargestellt, die mehrere Durchgangsöffnungen aufweist. Die Durchgangsöffnungen sind derart gestaltet, dass diese eine zentrale Bohrung mit einer zentralen Achse zeigen, wobei zusätzlich radial von der zentralen Achse beabstandet, mehrere (mindestens 3) weitere Ausnehmungen vorgesehen sind, die in einer um die zentralen Achse angeordneten sich zur Unterseite der Knochenplatte hin verjüngenden Innenmantelfläche angeordnet sind. Die Innenmantelfläche weist eine dreidimensionale Struktur auf.

Aus der DE 20309361U1 (KÖNIGSEE Implantate und Instrumente zur Ostheosynthese GmbH) 18.09.2003 sind Knochenplatten bekannt, die Bohrungen in der Ausbildung von Durchgangsöffnungen zur Aufnahme von Knochenschrauben aufweisen. Mindestens einige diese Bohrungen sind mit einem Innengewinde versehen, das konisch ausgebildet ist. Die Durchgangsöffnung ist derart ausgestaltet, dass diese aus mindestens zwei solcher Bohrungen besteht, die zueinander versetzt angeordnet sind und einander überlappen. Die Bohrungen sind nicht senkrecht Längserstreckung der Knochenplatte ausgeführt, sondern in definierten Winkeln.

### Aufgabe der Erfindung

Aufgabe der Erfindung ist es, eine Platte oder ein einfaches Schraubenverschlusssystem, bestehend aus Schraube und Platte zur Befestigung an Knoch bereitzustelen, mit dem polyaxial in Bezug auf die Platte beziehungsweise die Oberfläche des Knochens eine Verschraubung durchgeführt und eine Sicherung der Schraube gegen ungewolltest Lösen herbeigeführt werden kann.

### Lösung der Aufgabe

Der Grundgedanke der Lösung besteht darin, dass die Platte mindestens zwei Durchgangsbohrungen aufweist, wobei jede Durchgangsbohrung durch einen Mittelpunkt und durch einen Radius bestimmt ist. Die Durchgangsbohrungen selbst sind zueinander versetzt angeordnet und schneiden einander derart, dass sich Schnittlinien und/oder Schnittflächen in Richtung der Dicke der Platte ergeben, wobei diese Schnittlinien mit dem Gewinde des Schraubenkopfes zusammenwirken. Die Schnittlinien und/oder Schnittflächen erstrecken sich über die gesamte Dicke der Platte. Es findet somit eine plastische Verformung zwischen dem Schraubenkopf der eindrehenden Schraube und den Schnittlinien und/oder Schnittflächen statt, die zu einem Kraft- und Formschluss zwischen Platte und Schraube führen.

### Vorteile der Erfindung

Einer der wesentlichen Vorteile der Erfindung besteht darin, dass trotz der polyaxialen Einsatzmöglichkeit der Schrauben die Platte kein Gewinde aufweisen muss. Allein durch die Erstellung der Durchgangsbohrungen in der Platte werden Vorsprünge beziehungsweise Schnittlinien erzeugt, die ein insbesondere polyaxiales Einschrauben der Schraube in die Platte ermöglichen. Durch die hervorgerufene plastische Verformung wird ebenfalls erreicht, dass ein Lösen der Schraube aus der Platte nur noch mit erhöhter Kraftaufwendung möglich ist. Ein ungewolltes Lösen ist nicht möglich.

Vorteilhafterweise sind mindestens drei Durchgangsbohrungen vorgesehen, die zueinander derart angeordnet sind, dass alle drei Durchgangsbohrungen sich schneiden. Dies bedeutet, dass die erste Durchgangsbohrung die zweite Durchgangsbohrung und die dritte Durchgangsbohrung, die zweite Durchgangsbohrung die erste und die dritte und wiederum die dritte Durchgangsbohrung die erste und die zweite Durchgangsbohrung schneidet. Es entstehen dadurch drei Schnittlinien, die sich in die Dicke und damit in die Tiefe der Platte erstrecken. In der Mitte entsteht zentrisch eine Durchgangsöffnung, deren Radius durch den Abstand von einer Schnittlinie zum Mittelpunkt der Durchgangsöffnung bestimmt ist.

Eine bevorzugte Ausführungsform sieht vor, dass ausgehend von einer Zentralbohrung ein symmetrische Aufbau der Durchgangsbohrungen erfolgt. Hierfür sind die Durchgangsbohrungen, die in der Platte vorgesehen sind, derart angeordnet, dass deren jeweiligen Mittelpunkte auf einem Kreisbogen ausgehend von dem Mittelpunkt der Zentralbohrung angeordnet sind.

Ein wesentlicher Vorteil ist darin zu sehen, dass keine definierte Ober- und Unterseite der Knochenplatte vorgesehen ist. Dies bedeutet, dass die Platte unabhängig von ihrer Lage funktionsgerecht verwendet werden kann.

Alle vorgenannten Bohrungen einer besonderen Ausführungsform sind zylindrisch. Dies bedeutet, dass der Durchmesser der Durchgangsbohrungen und damit auch der Durchgangsöffnung über die Dicke der Platte gleichbleibend ist.

Die durch das Schneiden der jeweiligen Durchgangsbohrung entstehenden Schnittlinien beziehungsweise Schnittflächen sind derart ausgebildet, dass sie in Draufsicht auf die Platte zu dem jeweiligen Zentrum der Durchgangsöffnung hin spitz ausgebildet sind. Sie weisen einen Querschnitt auf, der derart ausgebildet ist, dass dieser von den Wandungen der Durchgangsbohrung breit ausgehend in Richtung Zentrum der Durchgangsöffnung spitz zuläuft. Bei einem Ausführungsbeispiel, bei dem die Durchgangsbohrungen jeweils senkrecht zur Platte ausgeübt werden, ist der Querschnitt über die Tiefe der Platte der jeweiligen Schnittlinie beziehungsweise Schnittfläche konstant. Dies bedeutet, dass bereits beim Aufsetzen der Schraube auf die erfindungsgemässe Platte mit der entsprechenden erfindungsgemässen Ausgestaltung der Durchgangsöffnung bereits mit der ersten Drehung in Eingriff kommt, um ein besseres Eindrehen zu ermöglichen, kann auch vorgesehen sein, dass der die jeweiligen Durchgangsbohrungen und damit auch die gesamte Durchgangsöffnung angesenkt wird. Dadurch kann die Schraube zunächst in die Durchgangsöffnung eingeführt werden, dort entsprechend platziert werden und erst mit einem weiteren Schritt dann in Eingriff mit der jeweiligen Schnittlinie beziehungsweise Schnittfläche gebracht werden.

Werden die Durchgangsbohrungen schräg, dass heisst nicht senkrecht zu der Längserstreckung der Platte ausgeführt, so entsehen ebenfalls schräge in die Tiefe der Platte sich erstreckenden Schnittlinien beziehungsweise Schnittflächen. Werden diese in unterschiedlichen Winkeln noch ausgeführt, so entstehen konische Ausbildungen.

Die Schnittlinien weisen dann an ihren freien Enden eine jeweils sehr geringe Dicke auf. Dadurch ist es möglich, dass das Gewinde des Schraubenkopfes in diesen Bereichen sich einfach einschneiden kann, da der Widerstand aufgrund der geringen Dicke gering ist. Die in Draufsicht V-förmige Ausbildung der Bereiche der Schnittlinien sind derart, dass mit zunehmenden Abstand von dem freien Ende der Schnittlinie der Querschnitt dicker wird. Dies ist insbesondere dann gegeben, wenn die Schraubenköpfe konisch gestaltet sind, wird mit zunehmenden Eindrehen der Schraube ein grösserer Querschnitt von dem Gewinde der Schraube durchdrungen. Dadurch findet eine stärke plastische Verformung statt.

Bei einem bevorzugten Ausführungsbeispiel ist vorgesehen, dass nach Herstellung einer zentralen Bohrung die Durchgangsbohrungen in Dickenrichtung der Platte nicht zylindrisch ausgestaltet sind, sondern konisch. Die Konizität ist derart ausgestaltet, dass sich die Durchgangsbohrung von der Einschraubeinrichtung zu dem Knochen hin verjüngt. Dies wird vorteilhafterweise durch die Verwendung eines konischen Fräsers erreicht. Vorzugsweise ist deren Symmetrieachse parallel zur Achse der Zentralbohrung und auch senkrecht zur Platte selbst. Die Mittelpunkte der Durchgangsbohrungen werden derart ausgewählt, dass deren Radius, der vorzugsweise bei allen Durchgangsbohrungen der gleiche ist, den Durchmesser der Zentralbohrung schneiden, so dass eine Durchgangsöffnung entsteht, die grösser als der Durchmesser der Zentralbohrung ist und an ihrer Aussenwandung eine Vielzahl von konischen Wandungen aufweist. Die einzelnen Wandungen sind konisch ausgebildet und verjüngen sich in Einschraubrichtung. Dadurch entstehen in Einschraubrichtung zunächst Schnittlinien (da der Durchmesser der einzelnen Durchgangsbohrungen gross ist), wohingegen mit zunehmender Tiefe (in Einschraubrichtung) der Durchmesser der Durchgangsbohrung aufgrund der vorgesehenen Konizität sich verringert und daher Schnittflächen zwischen den einzelnen Durchgangsbohrungen entstehen.

Es wurde festgestellt, dass die Anzahl der Durchgangsbohrungen nahezu proportional zur Einschraubkraft der Schraube ist. Dies bedeutet, dass bei einer grösseren Anzahl von Durchgangsbohrungen (beispielsweise 15) die Einschraubkraft im Vergleich zu einer geringeren Anzahl von Durchgangsbohrungen (beispielsweise 5) geringer ist.

Grundsätzlich ist es vorgesehen, beim Eindrehen der Schraube in die Platte mit zunehmender Einschraubtiefe mehr Kraft aufzuwenden. Dies liegt darin begründet, dass zunächst eine Interaktion, vorzugsweise eine Umformung mit den Schnittlinien stattfindet. Die mit der Schraube in Eingriff vorgesehene Linie beziehungsweise Volumen ist gering. Geht die Schnittlinie in eine Schnittfläche über, so wird das von der Schraube umzuformende Volumen grösser und damit muss mehr Kraft aufgebracht werden. Dadurch wird aber auch erreicht, dass eine sichere, nicht lösbare Verbindung zwischen der Platte und der Schraube entsteht.

Eine bevorzugte Weiterbildung sieht vor, zunächst die Zentralbohrung mit konischem Querschnitt zu erstellen. Dabei wird eine definierte Konizität gewählt. Die Durchgangsbohrungen, die kleeblattartig in Bezug auf diese Zentralbohrung folgen, weisen ebenfalls eine Konizität auf, aber im Gegensatz zur Zentralbohrung eine andere. Dadurch entstehen im entfernten Einschraubbereich innerhalb der Durchgangsöffnung keine Schnittlinien, sondern Schnittflächen.

Vorzugsweise weisen Schraubenkopf und -schaft denselben Durchmesser auf. Dies bringt den Vorteil mit sich, dass die Schraube einfach herstellbar ist.

Alternativ kann auch vorgesehen sein, dass der Schraubenkopf zylinderförmig ausgebildet ist und eine andere Gewindesteigung als der übrige Schaft aufweist.

Eine weitere Alternative kann vorsehen, dass der Schraubenkopf konisch ausgebildet ist und so sich an die Ausgestaltung der Durchgangsbohrung anpasst.

Der Schraubenschaft selbst kann auch konisch ausgebildet sein, um so den Kraftaufwand, insbesondere beim Eindrehen der Schraube in den Knochen, zu verringern.

Eine weitere alternative Ausführungsform sieht vor, den Schraubenkopf zumindest teilweise kugelig auszugestalten. Dadurch kann erreicht werden, dass ein grösserer Winkel, abweichend von der Senkrechten zur Platte erreicht wird, indem die Schraube noch innerhalb der Platte schräg (polyaxial) eingedreht werden kann.

Somit ist ein System vorgestellt worden, das aus ausschliesslich zwei Bauteilen besteht. Es ist nicht notwendig, zusätzliche Spreizelemente oder Unterlegelemente oder sonstige Materialien zu verwenden. Schraube und Platte können vorzugsweise aus demselben Material hergestellt werden. Um die plastische Verformung, insbesondere im Bereich der Vorsprünge, noch zu verbessern, kann vorgesehen sein, die Platte aus einem weicheren Material herzustellen als die Schraube selbst.

Einer der wesentlichen Vorteile des Systems liegt darin, dass die Platte selbst mit wenig Aufwand herzustellen ist. Die einfachste Ausführung besteht darin, drei Durchgangsbohrungen vorzusehen, die in einer bestimmten Anordnung zueinander auszubilden sind, so dass eine Durchgangsöffnung entsteht, in die die Schraube einführbar ist.

Die bevorzugte Ausbildung sieht vor, eine Zentralbohrung in der Platte vorzusehen, die von Durchgangsbohrungen geschnitten wird, wobei die Durchgangsbohrungen mit einem Kegelfräser hergestellt werden, der sich in Einschraubtiefe verjüngt. Die Anzahl der Durchgangsbohrungen sollte grösser als 5 sein. Eine bevorzugte Anzahl ist 15.

Eine weitere vorteilhafte Ausbildung kann vorsehen, dass die Schnittlinien oder/und Schnittflächen gehärtet sind und so gegenüber dem übrigen Material der Platte eine härtere Eigenschaft darstellen. Eine solche Härtung ist beispielsweise mit Laser durchführbar.

Weitere vorteilhafte Ausgestaltungen gehen aus den nachfolgenden Zeichnungen, der Beschreibung als auch den Ansprüchen hervor.

### Zeichnungen

Es zeigen:

Fig. 1 ein erstes Ausführungsbeispiel einer schematische Darstellung einer Durchgangsöffnung einer Platte als Teil des erfindungsgemässen Schraubenverschlusssystem;

Fig. 2 ein zweites Ausführungsbeispiel einer schematischen Darstellung einer Durchgangsöffnung einer Platte als Teil des erfindungsgemässen Schraubenverschlusssystem;

Fig. 3 ein drittes Ausführungsbeispiel einer schematische Darstellung einer Durchgangsöffnung einer Platte als Teil des erfindungsgemässen Schraubenverschlusssystem;

Fig. 4 eine schematische Darstellung des orthopädischen Schrauberiverschlusssystems im Schnitt, wobei die Schraube bereits in die Platte eingedreht ist;

Fig. 5A eine perspektivische Ansicht auf das Schraubenverschlusssystem gemäss Fig. 4;

Fig. 5B eine perspektivische Ansicht auf die Platte ohne Schrauben gemäss Fig. 5A;

Fig. 6 eine perspektivische Ansicht auf eine Platte mit einer Vielzahl Durchgangsöffnungen, wobei eine Durchgangsöffnung mit einer Schraube belegt ist;

Fig. 7 eine schematische Darstellung einer in einer Platte befindlichen Durchgangsöffnung, bestehend aus mehreren zylindrischen Durchgangsbohrungen, zur Erläuterung der Herstellung;

Fig. 8 eine weitere schematische Darstellung einer in einer Platte befindlichen Durchgangsöffnung, bestehend aus mehreren konischen Durchgangsbohrungen;

Fig. 9 eine weitere schematische Darstellung eines anderen Ausführungsbeispiels einer in einer Platte befindlichen Durchgangsöffnung, bestehend aus mehreren konischen Durchgangsbohrungen.

### Beschreibung der Ausführungsbeispiele

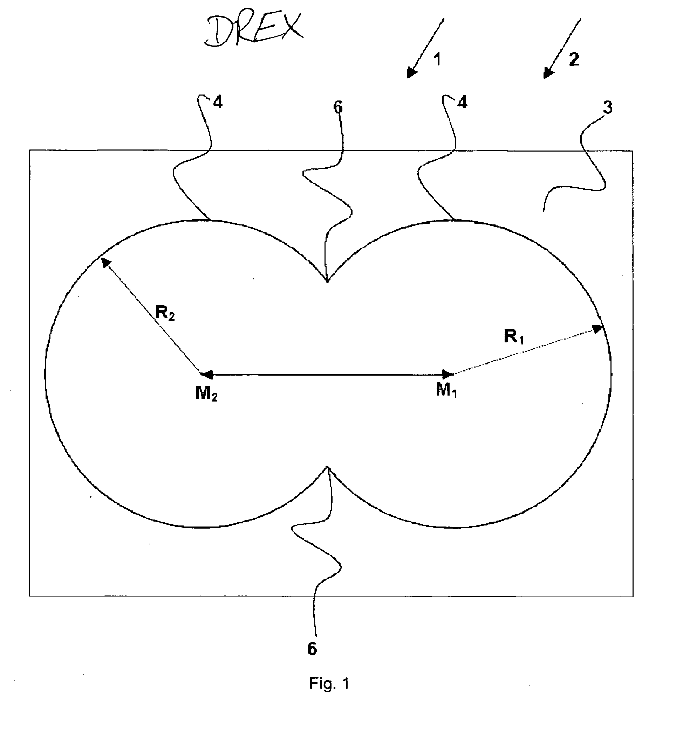

In den Fig. 1 bis 3 ist eine Auswahl von möglichen Durchgangsöffnungen 1 für ein orthopädisches Schraubenverschlusssystem 2 dargestellt. Zur Herstellung einer solchen erfindungsgemässen Durchgangsöffnung 1, angeordnet auf einer Platte 3, werden mindestens zwei Durchgangsbohrungen 4 erzeugt. Jede Durchgangsbohrung 4 ist durch einen Radius R₁, R₂ und einen Mittelpunkt M₁, M₂ bestimmt.

Gemäss einem ersten Ausführungsbeispiel, wie es in Fig. 1 dargestellt ist, besteht die Durchgangsöffnung 1 aus zwei Durchgangsbohrungen 4, deren beiden Mittelpunkte M₁, M₂ in einem.Abstand 5 zueinander angeordnet sind. Der Abstand 5 ist derart zu wählen, dass die beiden Radien R₁, R₂ der Durchgangsbohrungen 4 sich schneiden. Dadurch entstehen Schnittlinien 6, die sich in die Tiefe (Dicke) der Platte 3 erstrecken. Die Schnittlinien 6 sind derart ausgebildet, dass sie zum freien Raum der Durchgangsöffnung 1 hin spitz ausgebildet sind und sich von hier ausgehend mit zunehmender Entfernung eine dickere Wandstärke aufweisen. Dadurch ist es möglich, dass die so gebildeten freien Enden durch ein Eindrehen einer Schraube plastisch verformbar sind.

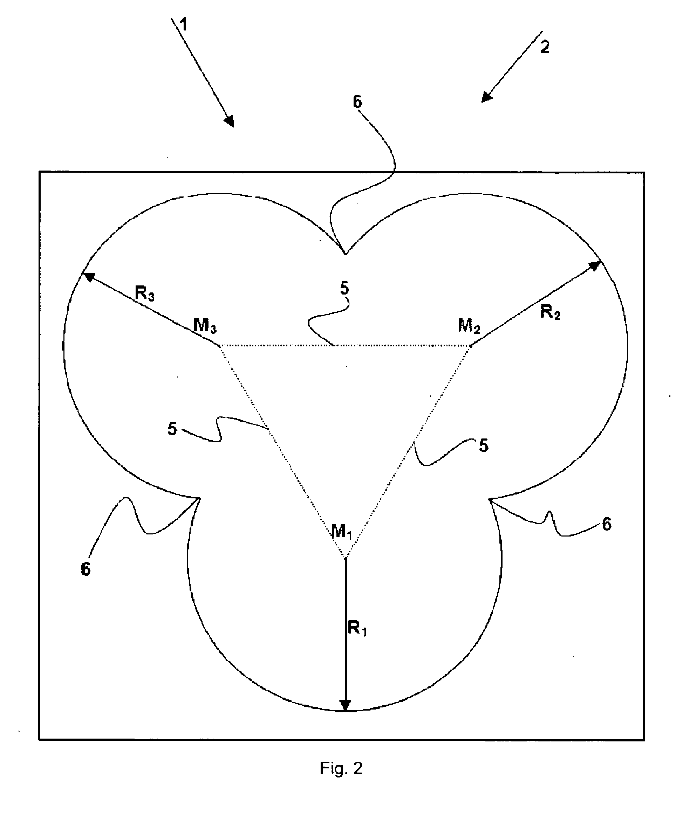

Gemäss einem weiteren Ausführungsbeispiel, wie es in Fig. 2 dargestellt ist, besteht die Durchgangsöffnung 1 aus drei Durchgangsbohrungen 4, deren Mittelpunkte M₁, M₂, M₃ jeweils in einem Abstand 5 voneinander angeordnet sind. Der jeweilige Abstand 5 ist derart zu wählen, dass die drei Radien R₁, R₂, R₃ der Durchgangsbohrungen 4 sich schneiden. Dadurch entstehen Schnittlinien 6, die sich in die Tiefe (Dicke) der Platte 3 erstrecken.

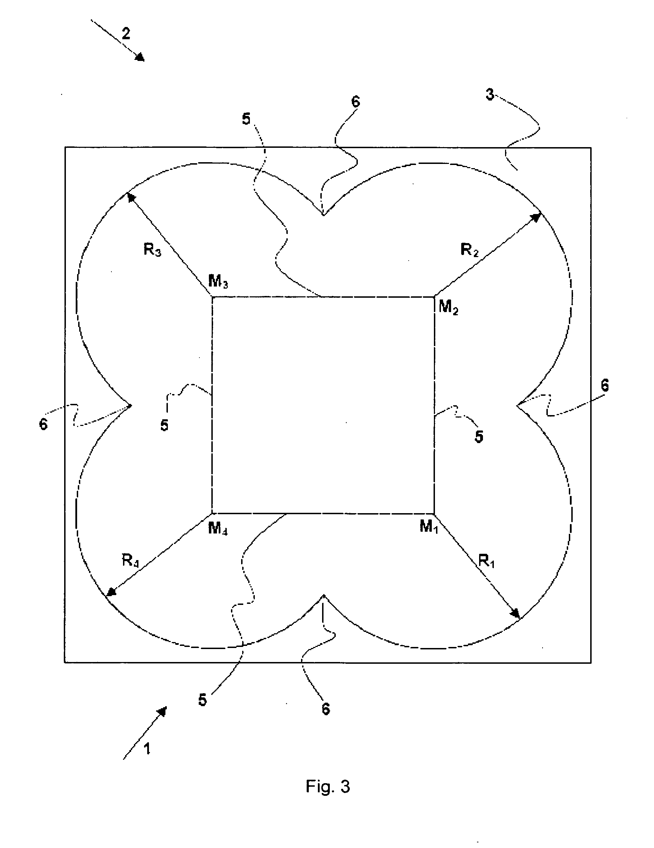

Gemäß einem weiteren Ausführungsbeispiel, wie es in Fig. 3 dargestellt ist, besteht die Durchgangsöffnung 1 aus vier Durchgangsbohrungen 4, deren Mittelpunkte M₁, M₂, M₃, M₄ jeweils in einem Abstand 5 voneinanderangeordnet sind. Der jeweilige Abstand 5 derart zu wählen, dass die vier Radien R₁, R₂, R₃, R₄ der Durchgangsbohrungen 4 sich schneiden
Dadurch entstehen Schnittlinien 6, die sich in die Tiefe (Dicke) der Platte 3 erstrecken.

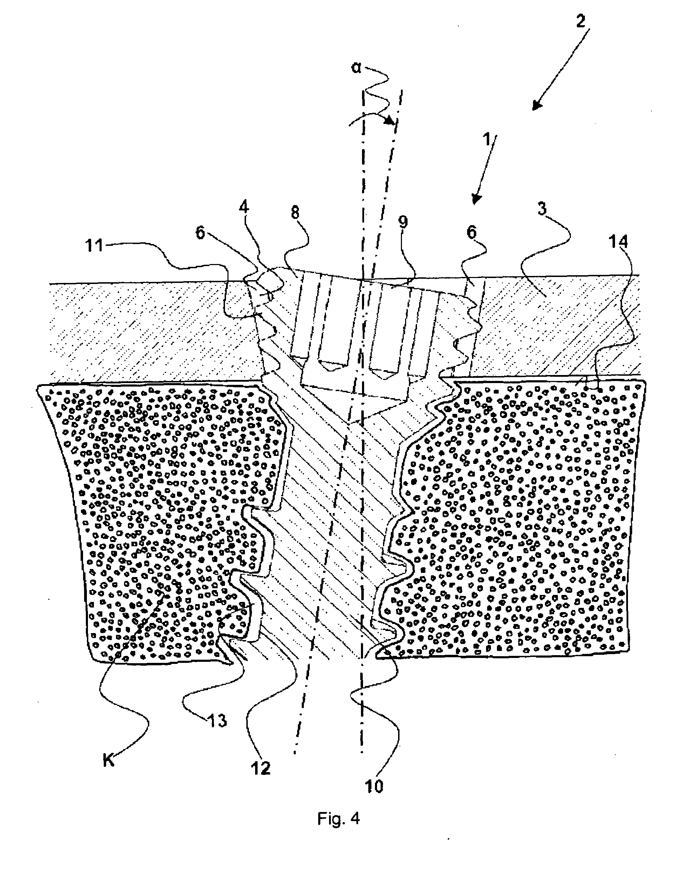

In den Fig. 4, 5A, 5B und 6 ist das orthopädische Schraubenverschlusssystem 2 dargestellt. Es besteht aus einer Schraube 8 und der Platte 3, wie sie beispielsweise in den Fig. 1 bis 3 dargestellt ist. Die Platte 3 dient zur Fixierung beispielsweise einer Fraktur. Die Schraube 8 selbst ist in einen Knochen K eingedreht. Die Platte 3 liegt mit ihrer Unterseite 14 auf der Aussenfläche des Knochens K auf (Fig. 4). Die Durchgangsöffnung 1, wie sie in den Fig. 1-3 exemplarisch dargestellt ist, dient zur Aufnahme der Schraube 8, die aus einem Schraubenkopf 9 und einem Schraubenschaft 10 besteht. Sowohl Schraubenkopf 9 als auch der Schraubenschaft 10 weisen jeweils ein Gewinde auf. Das Gewinde 11 des Schraubenkopfs 8 wirkt mit den Schnittlinien 6 zusammen. Das Gewinde 12 des Schraubenschafts 10 wirkt mit dem Knochen K zusammen. Im Knochen K selbst ist vorzugsweise bereits eine Bohrung 13 vorgesehen, deren lichte Weite geringer ist, als der Durchmesser des Schraubenschafts 10. Aufgrund dessen, dass die lichte Weite der Durchgangsöffnung 1 grösser ist als die des Durchmessers des Schraubenkopfs 9, ist es möglich, so wie auch in Fig. 4 dargestellt, die Schraube 8 nicht senkrecht zur Platte 3, sondern geneigt in einem beliebigen Winkel a anzuordnen.

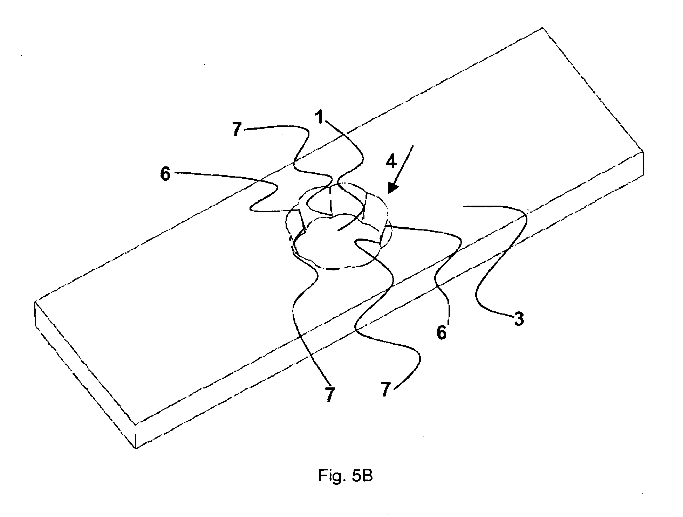

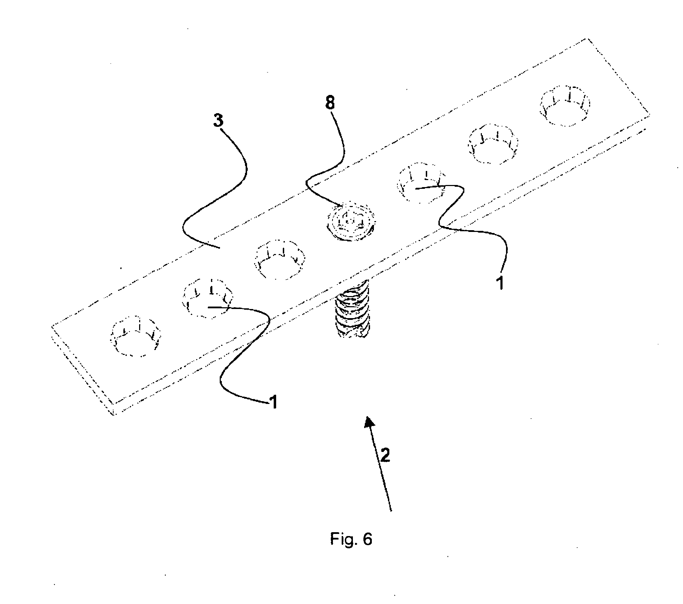

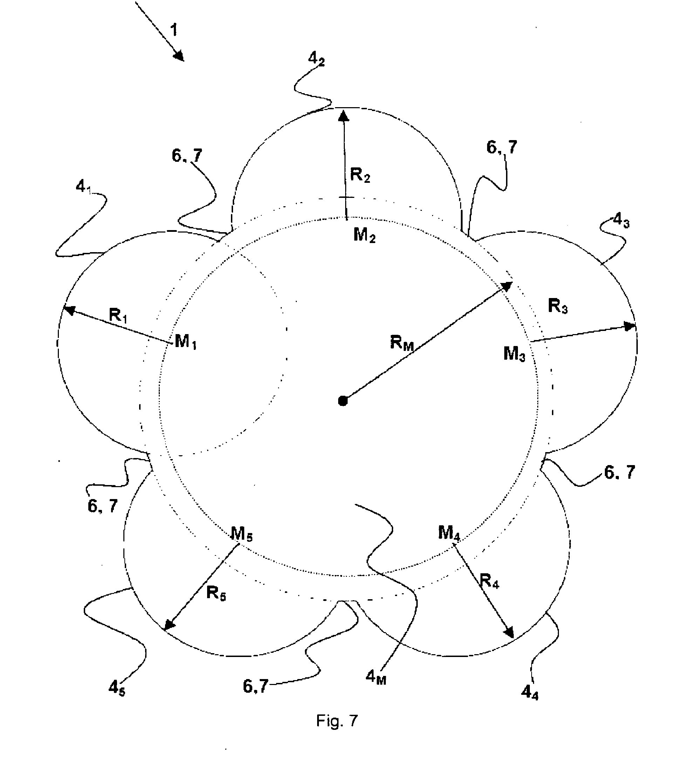

In Fig. 7 ist schematisch dargestellt, wie eine Durchgangsöffnung 1, wie sie in Fig. 5B und Fig. 6 dargestellt ist, konstruiert wird. Zunächst wird der Mittelpunkt M der Durchgangsöffnung 1 festgelegt. Anschliessend wird ein Radius RM festgelegt. Dieser dient dazu, dass alle auf dem Kreis mit dem Radius RM liegenden weiteren Mittelpunkte M₁, M₂, M₃, M₄, M₅ den gleichen Abstand zum Mittelpunkt R_{M} aufweisen. Anschliessend wird der Kreis mit dem Mittelpunkt R_{M} derart aufgeteilt, dass eine beliebige Anzahl von weiteren Mittelpunkten für weitere Kreise im gleichen Abstand zueinander angeordnet sind. Hier in Fig. 6 wird der Kreis derart aufgeteilt, dass die Mittelpunkte M₁, M₂, M₃, M₄, M₅ den gleichen Abstand zueinander haben.

Zunächst ist eine Zentralbohrung 4_{M} mit dem Mittelpunkt M und dem Radius R_{M} zu erzeugen. Die Zentralbohrung 4M bei dem dargestellten Ausführungsbeispiel ist zylinderförmig und gleichförmig über die gesamte Dicke der Platte 3. Anschliessend wird die erste Durchgangsbohrung 4₁ mit einem Mittelpunkt M₁ und einem Radius R₁ erzeugt. Sobald die Bohrung erstellt ist, ist eine zylinderförmige Durchgangsbohrung 4₁ entstanden. Bei den Punkten M₂ bis M₅ wird dann wiederum eine Durchgangsbohrung 4₂...4₅ angesetzt und zwar mit dem Radien R₂...R₅, wobei die Radien R₁...R₅ identisch sind. Dadurch entsteht die kleeblättrige Form mit den jeweiligen Schnittlinien 6, die mit dem Schraubenkopfes 9 zusammenwirken. Diese Ausführungsform bietet die Möglichkeit, nach Herstellung einer Zentralbohrung 4M eine Vielzahl von Durchgangsbohrungen 4ₓ mit Radius Rₓ und Mittelpunkt Mₓ bereitzustellen. Dabei ist x Element der natürlichen Zahlen von 1 bis unendlich.

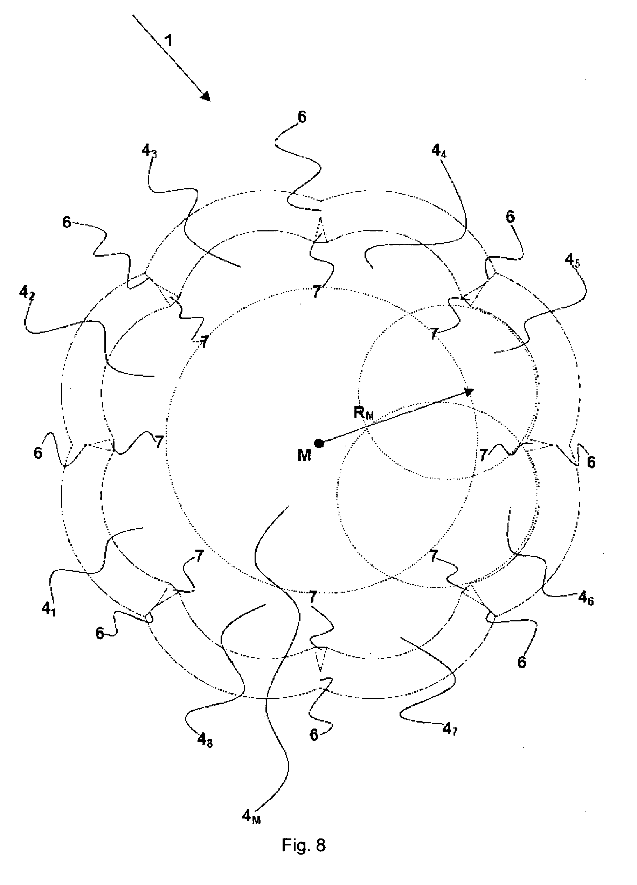

Fig. 8 zeigt eine weitere Ausbildungsform der Ausführung gemäss Fig. 7. Zunächst ist eine Zentralbohrung 4_{M} mit dem Mittelpunkt M und dem Radius R_{M} zu erzeugen. Die Zentralbohrung 4_{M} bei dem dargestellten Ausführungsbeispiel ist konisch ausgestaltet und verjüngt sich über die Dicke der Platte 3 ausgehend von der Oberseite der Platte. Die definierte Konizität, dass heisst eine Veränderung des Durchmessers des Konus in Abhängigkeit von der Dicke der Platte, ist derart gewählt, dass sich der Durchmesser in Schraubrichtung der Schraube verjüngt. Aufgrund dessen, dass die weiteren Durchgangsbohrungen die Zentralbohrung 4_{M} vollständig schneiden, reicht es aus, die Zentralbohrung 4_{M} zylindrisch auszugestalten.

Anschliessend wird die erste Durchgangsbohrung 4₁ mit einem Mittelpunkt M₁ und einem Radius R₁ erzeugt. Die Durchgangsbohrung 4₁ bei dem dargestellten Ausführungsbeispiel ist konisch ausgestaltet und verjüngt sich über die Dicke der Platte 3. Die Konizität, dass heisst eine Veränderung des Durchmessers des Konus in Abhängigkeit von der Dicke der Platte, ist derart gewählt, dass sich der Durchmesser in Schraubrichtung der Schraube verjüngt. Sobald die Bohrung erstellt ist, ist eine konische Durchgangsbohrung 4₁ entstanden. Bei den Mittelpunkten M₂ bis M₅ wird dann wiederum eine Durchgangsbohrung 4₂...4₅ angesetzt und zwar mit dem Radien R₂...R₅, wobei die Radien R₁...R₅ und die Konizitäten identisch sind. Dadurch entsteht die in Draufsicht kleeblättrige Form mit den jeweiligen Schnittlinien 6, die mit dem Schraubenkopfes 9 zusammenwirken. Zusätzlich entstehen aufgrund der Konizitäten Durchgangsbohrungen bevorzugt in Verlängerung der Schnittlinie 6 zusätzliche Schnittflächen 7. Dies bedeutet, dass die Schnittlinien 6 mit zunehmender Tiefe in Schnittflächen 7 übergehen. Bei den in den Fig. 5B und 8 dargestellten Ausführungsbeispielen sind die Schnittflächen 7 auf der dem Knochen zugewandten Seite der Platte 3 und nehmen ca. 1/3 der Tiefe der Platte ein.

Die Anzahl der Durchgangsbohrungen ist frei zu wählen. Es wurde festgestellt, dass eine Vielzahl von Durchgangsbohrungen die Einschraubkraft der Schraube verringern. Je mehr Durchgangsbohrungen vorhanden sind, desto grösser ist zwar die Anzahl der Schnittflächen und Schnittlinien. Dennoch ist die vorhandene (aus der Draufsicht gesehen) Dicke der Schnittlinien und -flächen geringer.

Im Gegensatz zu Fig. 8 sind zum einen mehrere Durchgangsbohrungen vorgesehen und die Durchgangsbohrungen 4₁...4₈ sind konisch, wobei diese Durchgangsbohrungen sich zum Knochen K hin verjüngen.

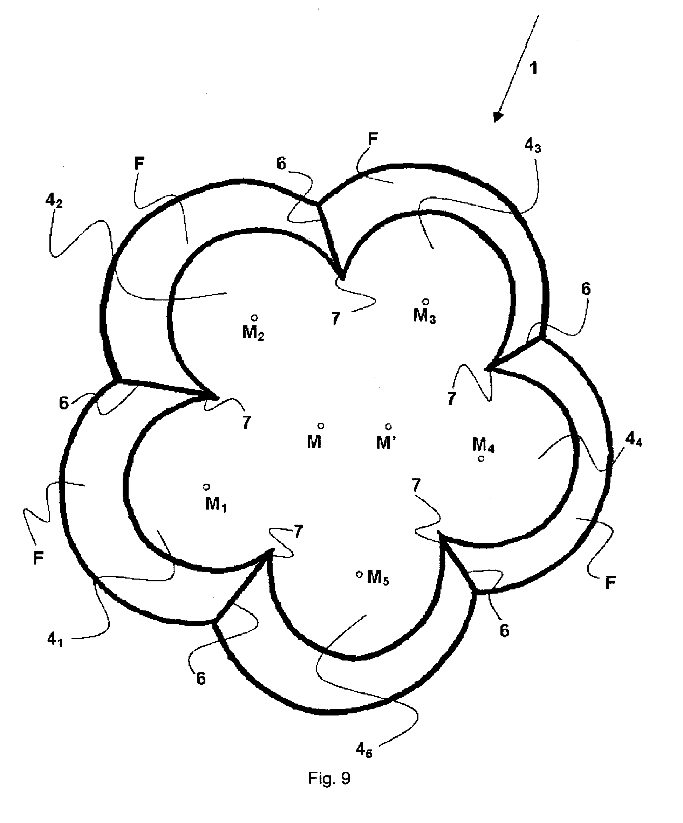

Fig. 9 zeigt eine weitere Ausführungsform, wobei die konischen Flächen im Gegensatz zu Fig. 8 asymmetrisch ausgebildet sind. Die Asymmetrie wird dadurch erreicht, dass an sich zwei zentrale Durchgangsbohrungen mit jeweils einem Mittelpunkt M, M' vorgesehen sind. Von diesen jeweiligen Mittelpunkten ausgehend erstrecken sich im unterschiedlichen Abstand die jeweiligen Mittelpunkt Mₓ (M₁...M₅) in unterschiedlicher Länge beziehungsweise unterschiedlichem Abstand. Dadurch entsteht in Draufsicht eine rosettenartige Ausbildung der erfindungsgemässen Durchgangsöffnung 1 eine leicht verschobene Rosettenausbildung, die unterschiedlich lange schräge Auflageflächen F bildet. Die in Draufsicht erkennbaren Schnittlinien 6 gehen dann wie bei den vorherigen Beispielen ebenfalls, mit zunehmender Tiefe in Schnittflächen über.

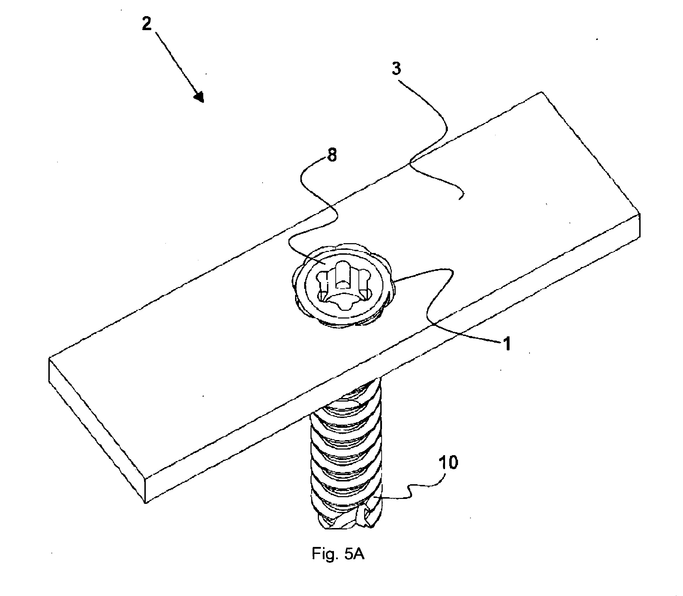

Insbesondere der in Fig. 5A bis 9 sind Ausführungen der erfindungsgemässen Durchgangsöffnung gezeigt, die besonders ausgestaltet sind. Durch die konische Ausbildung der jeweiligen Durchgangsöffnungen entsteht zunächst in Schraubrichtung eine Schnittlinie, die sich mit zunehmender Tiefe in Richtung des Knochens zu einer Schnittfläche ausbildet. Dieses ist insbesondere in Fig. 5B deutlich zu erkennen. Dies bringt den Vorteil mit sich, dass zunächst nur wenige Gewindegänge beim Einschrauben mit der Schnittlinie zusammenwirken und dort eine geringe plastische Verformung ausüben und je tiefer die Schraube hineingedreht wird, desto grösser muss die Interaktion zwischen der Schnittlinie beziehungsweise Schnittfläche und dem Gewinde der jeweiligen Schraube sein. Dadurch wird erreicht, dass aufgrund der plastischen Umformung eine nahezu nicht lösbare Verbindung entsteht.

Alternativ zu dieser konischen Ausbildung ist auch vorgesehen, die Durchgangsbohrungen, die um die zentrale Bohrung herum angeordnet sind, jeweils in einem Winkel anzuordnen, sodass sich die Durchgangsöffnung nach unten hin ebenfalls verjüngt. Damit ist es nicht notwendig, ausschliesslich konische Bohrungen herzustellen. Es ist daher auch möglich nachdem die zentrale Bohrung hergestellt worden ist, solche Ausbildungen bereitzustellen, wie sie in Fig. 5 bis 9 gezeigt sind. Durch die schräge Anordnung der jeweiligen Bohrer zur Herstellung der Durchgangsbohrung mit jeweiligem Mittelpunkt Mₓ und Radius Rₓ, obei x Element der natürlichen Zahlen 1 bis unendlich ist, ist es daher möglich, ein selbstschliessendes Schraubenverschlusssystem, gebildet aus Schraube und Platte zur Anwendung im orthopädischen Bereich.

Das orthopädische Schraubenverschlusssystem stellt somit eine wesentlichen Neuerung gegenüber dem Stand der Technik dar, da es ausschliesslich aus zwei Elementen besteht, aber Eigenschaften aufweist, die bisher nur mit mehreren Elementen in komplexer Bauweise bekannt ist. Durch die kleeblattähnliche Struktur der Durchgangsöffnung bestehen für die Schraube Einschraubmöglichkeiten in unterschiedlichen Winkeln. Dabei zeichnet sich das System dadurch aus, dass die Schraube gegen ungewolltes Lösen gesichert ist.

### Bezugszeichenliste

- 1: Durchgangsöffnung
- 2: Orthopädisches Fixierungssystem/Schraubenverschiusssystem
- 3: Platte
- 4: Durchgangsbohrungen
- 4M: Zentralbohrung
- 4x: Durchgangsbohrungen
- 5: Abstand
- 6: Schnittlinien
- 7: Schnittflächen
- 8: Schraube
- 9: Schraubenkopf
- 10: Schraubenschaft
- 11: Gewinde
- 12: Gewinde
- 13: Bohrung
- 14: Unterseite Platte
- Rx: Radius
- Mx: Mittelpunkt
- K: Knochen

## Patentansprüche

1. Orthopädisches Schraubenverschlusssystem, umfassend
- mindestens eine Schraube (8), bestehend jeweils aus einem Schraubenkopf (9) und einem Schraubenschaft (10), wobei der Schraubenkopf (9) mit einem Gewinde (11) versehen ist,
und
- mindestens eine Platte (3), die eine entsprechende Dicke aufweist, versehen mit mindestens einer Durchgangsöffnung (1), die geeignet ist, die Schraube (8) aufzunehmen, wobei
- die Platte (3) eine Durchgangsöffnung (1), gebildet aus mindestens zwei Durchgangsbohrungen (4) aufweist, wobei jede Durchgangsbohrung (4ₓ) durch einen Mittelpunkt (Mₓ) und durch einen Radius (Rₓ) bestimmt ist,
- die Durchgangsbohrungen (4ₓ) zueinander versetzt angeordnet sind,
- die Mittelpunkte (Mx) der Durchgangsbohrungen (4x) auf einem gemeinsamen Kreisbogen angeordnet sind
- die Durchgangsbohrungen (4x) einander derart schneiden, dass sich Schnittlinien (6) und/oder Schnittflächen (7) bilden, die sich in die Tiefe der Durchgangsöffnung (1) über die Dicke der platte (3) erstrecken, **dadurch gekennzeichnet, dass** die Schnittlinien (6) und/oder Schnittflächen (7) durch die Anordnung der Durchgangsbohrungen (4x) derart ausgebildet sind, dass sie in Draufsicht auf die Platte (3) zu dem Zentrum der Durchgangsöffnung (1) spitz ausgebildet sind zur Interaktion mit dem Schraubenkopf (9) der Schrauben (8).

2. Schraubenverschlusssystem nach Anspruch 1, **dadurch gekennzeichnet, dass** die Schnittlinien (6) und/oder Schnittflächen (7) sich in axialer Richtung der Durchgangsbohrungen (4ₓ) erstrecken.

3. Schraubenverschlusssystem nach einem der vorhergehenden Ansprüche, **dadurch gekennzeichnet, dass** bei drei oder mehr Durchgangsbohrungen (4ₓ) diese derart zueinander versetzt angeordnet sind, dass jede Durchgangsbohrung (4ₓ) alle übrigen Durchgangsbohrungen (4ₓ) mindestens einmal schneidet.

4. Schraubenverschlusssystem nach mindestens einem der vorhergehenden Ansprüche, **dadurch gekennzeichnet, dass** mindestens eine Durchgangsbohrung (4ₓ) konisch ausgestaltet ist.

5. Schraubenverschlusssystem nach Anspruch 4, **dadurch gekennzeichnet, dass** sich die Durchgangsbohrungen (4ₓ) in Einschraubrichtung verjüngen.

6. Schraubenverschlusssystem nach Anspruch 4 oder 5, **dadurch gekennzeichnet, dass** in Einschraubrichtung der Durchgangsöffnung (1) zunächst Schnittlinien (6) vorgesehen, die in Schnittflächen (7) übergehen.

7. Schraubenverschlusssystem nach Anspruch 1, **dadurch gekennzeichnet, dass** eine Durchgangsöffnung (1) durch Herstellung einer Zentralbohrung (4_{M}) mit einem Radius (R_{M}) um einen Mittelpunkt M und anschliessender weiterer Durchgangsbohrungen (4ₓ), die kleeblattartig um die Zentralbohrung (4_{M}) angeordnet sind, herstellbar ist.

8. Schraubenverschlusssystem nach mindestens einem der vorhergehenden Ansprüche, **dadurch gekennzeichnet, dass** der Schraubenkopf (8) konisch und sich zum Schraubenschaft (10) verjüngend ausgebildet ist.

9. Orthopädische Platte, insbesondere Knochenplatte bestehend aus einem plattenartigen Grundelement sowie in dem Grundelement angeordnete mindestens eine Durchgangsöffnung (1), die geeignet ist, eine Schraube (8) aufzunehmen, wobei
- die Platte (3) eine Durchgangsöffnung (1), gebildet aus mindestens zwei Durchgangsbohrungen (4ₓ) aufweist, wobei jede Durchgangsbohrung (4ₓ) durch einen Mittelpunkt (Mₓ) und durch einen Radius (Rₓ) bestimmt ist,
- die Durchgangsbohrungen (4ₓ) zueinander versetzt angeordnet sind,
- die Mittelpunkte (Mx) der Durchgangsbohrungen (4x) auf einem gemeinsamen Kreisbogen angeordnet sind,
die Durchgangsbohrungen (4x) einander derart schneiden, dass sich Schnittlinien (6) und/oder Schnittflächen (7) bilden, die sich in die Tiefe der Durchganpsöffung über die Dicke der platte erstrecken **dadurch gekennzeichnet, dass** die Schnittlinien (6) und/oder Schnittflächen (7) durch die Anordnung der Durchgangsbohrungen (4x) derart ausgebildet sind, dass sie in Draufsicht auf die Platte (3) zu dem Zentrum der Durchgangsöffnung (1) spitz ausgebildet sind zur Interaktion mit dem Schraubenkopf (9) der Schraube (8).

10. Platte nach Anspruch 9, **dadurch gekennzeichnet, dass** der jeweilige Mittelpunkt (Mₓ) der Durchgangsbohrung (4ₓ) auf einem gemeinsamen Kreis angeordnet ist.

11. Platte nach Anspruch 9 oder 10, **dadurch gekennzeichnet, dass** die jeweilige Durchgangsbohrung (4ₓ) eine Konizität aufweist.

12. Platte nach Anspruch 1 1 , **dadurch gekennzeichnet, dass** sich die Durchgangsbohrung (4ₓ) in Einschraubrichtung verjüngt.

## Claims

1. Orthopedic screw fastener system, comprising
- at least one screw (8), consisting in each case of a screw head (9) and of a screw shank (10), the screw head (9) being provided with a thread (11),
and
- at least one plate (3), which has a suitable thickness, provided with at least one through-opening (1) which is able to receive the screw (8),
- the plate (3) having a through-opening (1), formed by at least two through-bores (4), each through-bore (4ₓ) being defined by a midpoint (Mₓ) and by a radius (Rₓ),
- the through-bores (4ₓ) being offset relative to each other,
- the midpoints (Mₓ) of the through-bores (4ₓ) being arranged on a common arc of a circle,
- the through-bores (4ₓ) intersecting each other in such a way that intersection lines (6) and/or intersection surfaces (7) form, which extend into the depth of the through-opening (1) over the thickness of the plate (3), **characterized in that** the intersection lines (6) and/or intersection surfaces (7) are configured, by arrangement of the through-bores (4ₓ), in such a way that, in a plan view of the plate (3), they taper toward the center of the through-opening (1) for interaction with the screw head (9) of the screws (8).

2. Screw fastener system according to Claim 1, **characterized in that** the intersection lines (6) and/or intersection surfaces (7) extend in the axial direction of the through-bores (4ₓ).

3. Screw fastener system according to one of the preceding claims, **characterized in that**, in the case of three or more through-bores (4ₓ), these are offset relative to one another in such a way that each through-bore (4ₓ) intersects all the other through-bores (4ₓ) at least once.

4. Screw fastener system according to at least one of the preceding claims, **characterized in that** at least one through-bore (4ₓ) has a conical shape.

5. Screw fastener system according to Claim 4, **characterized in that** the through-bores (4ₓ) narrow in the screwing-in direction.

6. Screw fastener system according to Claim 4 or 5, **characterized in that**, in the screwing-in direction of the through-opening (1), intersection lines (6) are first provided, which merge into intersection surfaces (7).

7. Screw fastener system according to Claim 1, **characterized in that** a through-opening (1) can be produced by producing a central bore (4_{M}) with a radius (R_{M}) about a midpoint M, and subsequent further through-bores (4ₓ) that are arranged like a cloverleaf around the central bore (4_{M}).

8. Screw fastener system according to at least one of the preceding claims, **characterized in that** the screw head (9) is conical and narrows toward the screw shank (10).

9. Orthopedic plate, in particular a bone plate, consisting of a plate-like main element and at least one through-opening (1) arranged in the main element, the through-opening being adapted to hold a screw (8),
- the plate (3) having a through-opening (1), formed by at least two through-bores (4ₓ), each through-bore (4ₓ) being defined by a midpoint (Mₓ) and by a radius (Rₓ),
- the through-bores (4ₓ) being offset relative to each other,
- the midpoints (Mₓ) of the through-bores (4ₓ) being arranged on a common arc of a circle,
- the through-bores (4ₓ) intersecting each other in such a way that intersection lines (6) and/or intersection surfaces (7) form, which extend into the depth of the through-opening over the thickness of the plate, **characterized in that** the intersection lines (6) and/or intersection surfaces (7) are configured, by the arrangement of the through-bores (4ₓ), in such a way that, in a plan view of the plate (3), they taper toward the center of the through-opening (1) for interaction with the screw head (9) of the screw (8).

10. Plate according to Claim 9, **characterized in that** the respective midpoint (Mₓ) of the through-bore (4ₓ) is arranged on a common circle.

11. The plate according to Claim 9 or 10, **characterized in that** the respective through-bore (4ₓ) has a conicity.

12. The plate according to Claim 11, **characterized in that** the through-bore (4ₓ) narrows in the screwing-in direction.

## Revendications

1. Système de fermeture à vis orthopédique, comprenant :
- au moins une vis (8), constituée dans chaque cas d'une tête de vis (9) et d'une tige de vis (10), la tête de vis (9) étant pourvue d'un filetage (11),
et
- au moins une plaque (3) qui présente une épaisseur correspondante, pourvue d'au moins une ouverture de passage (1) qui est appropriée pour recevoir la vis (8),
- la plaque (3) présentant une ouverture de passage (1) formée d'au moins deux alésages de passage (4), chaque alésage de passage (4ₓ) étant déterminé par un centre (Mₓ) et un rayon (Rₓ),
- les alésages de passage (4ₓ) étant décalés l'un par rapport à l'autre,
- les centres (Mₓ) des alésages de passage (4ₓ) étant disposés sur un arc de cercle commun,
- les alésages de passage (4ₓ) se coupant de telle sorte que des lignes de coupe (6) et/ou des surfaces de coupe (7) se forment, lesquelles s'étendent dans la profondeur de l'ouverture de passage (1) sur l'épaisseur de la plaque (3), **caractérisé en ce que** les lignes de coupe (6) et/ou les surfaces de coupe (7) sont réalisées par l'agencement des alésages de passage (4ₓ) de telle sorte qu'elles soient réalisées en vue de dessus sur la plaque (3) en pointe vers le centre de l'ouverture de passage (1) en vue de l'interaction avec la tête de vis (9) des vis (8).

2. Système de fermeture à vis selon la revendication 1, **caractérisé en ce que** les lignes de coupe (6) et/ou les surfaces de coupe (7) s'étendent dans la direction axiale des alésages de passage (4ₓ).

3. Système de fermeture à vis selon l'une quelconque des revendications précédentes, **caractérisé en ce que** dans le cas de trois alésages de passage (4ₓ) ou plus, ceux-ci sont décalés les uns par rapport aux autres de telle sorte que chaque alésage de passage (4ₓ) coupe au moins une fois tous les autres alésages de passage (4ₓ).

4. Système de fermeture à vis selon au moins l'une quelconque des revendications précédentes, **caractérisé en ce qu'**au moins un alésage de passage (4ₓ) est configuré sous forme conique.

5. Système de fermeture à vis selon la revendication 4, **caractérisé en ce que** les alésages de passage (4ₓ) se rétrécissent dans la direction de vissage.

6. Système de fermeture à vis selon la revendication 4 ou 5, **caractérisé en ce que** des lignes de coupe (6) sont d'abord prévues dans la direction de vissage de l'ouverture de passage (1), lesquelles se prolongent par des surfaces de coupe (7).

7. Système de fermeture à vis selon la revendication 1, **caractérisé en ce qu'**une ouverture de passage (1) peut être créée en formant un alésage central (4_{M}) avec un rayon (R_{M}) autour d'un centre M, puis d'autres alésages de passage (4ₓ), qui sont disposés en forme de feuille de trèfle autour de l'alésage central (4_{M}).

8. Système de fermeture à vis selon au moins l'une quelconque des revendications précédentes, **caractérisé en ce que** la tête de vis (9) est réalisée sous forme conique et se rétrécit vers la tige de vis (10).

9. Plaque orthopédique, notamment plaque à os constituée d'un élément de base en forme de plaque ainsi que d'au moins une ouverture de passage (1) disposée dans l'élément de base, qui est appropriée pour recevoir une vis (8),
- la plaque (3) présentant une ouverture de passage (1) formée d'au moins deux alésages de passage (4ₓ), chaque alésage de passage (4ₓ) étant déterminé par un centre (Mₓ) et un rayon (Rₓ),
- les alésages de passage (4ₓ) étant décalés l'un par rapport à l'autre,
- les centres (Mₓ) des alésages de passage (4ₓ) étant disposés sur un arc de cercle commun,
- les alésages de passage (4ₓ) se coupant de telle sorte que des lignes de coupe (6) et/ou des surfaces de coupe (7) se forment, lesquelles s'étendent dans la profondeur de l'ouverture de passage sur l'épaisseur de la plaque, **caractérisée en ce que** les lignes de coupe (6) et/ou les surfaces de coupe (7) sont réalisées par l'agencement des alésages de passage (4ₓ) de telle sorte qu'elles soient réalisées en vue de dessus sur la plaque (3) en pointe vers le centre de l'ouverture de passage (1) en vue de l'interaction avec la tête de vis (9) des vis (8).

10. Plaque selon la revendication 9, **caractérisée en ce que** le centre respectif (Mₓ) des alésages de passage (4ₓ) est disposé sur un cercle commun.

11. Plaque selon la revendication 9 ou 10, **caractérisée en ce que** l'alésage de passage (4ₓ) respectif présente une conicité.

12. Plaque selon la revendication 11, **caractérisée en ce que** l'alésage de passage (4ₓ) se rétrécit dans la direction de vissage.
